(19) Europäisches Patentamt / European Patent Office / Office européen des brevets

(11) **EP 3 527 644 A1**

(12) **EUROPEAN PATENT APPLICATION**

(43) Date of publication:
**21.08.2019 Bulletin 2019/34**

(21) Application number: **19168881.1**

(22) Date of filing: **07.07.2015**

(51) Int Cl.:
*C10G 29/20* (2006.01)    *B01J 29/08* (2006.01)
*C07C 2/58* (2006.01)    *B01J 35/02* (2006.01)
*B01J 38/56* (2006.01)    *B01J 38/58* (2006.01)
*B01J 29/12* (2006.01)    *B01J 29/70* (2006.01)
*B01J 29/90* (2006.01)    *B01J 29/068* (2006.01)
*B01J 29/74* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.07.2014 US 201462021433 P**

(62) Document number(s) of the earlier application(s) in accordance with Art. 76 EPC:
**15741953.2 / 3 167 026**

(71) Applicant: **Albemarle Europe Sprl.**
**1348 Louvain-la-Neuve (BE)**

(72) Inventors:
• **VAN BROEKHOVEN, Emanuel Hermanus**
  **1141 Monnickendamm (NL)**
• **VAN LOEVEZIJN, Arnold**
  **1326 Almere (NL)**
• **BAKKER, Richard Hendrik Marc**
  **2031 Harlem (NL)**

(74) Representative: **SSM Sandmair**
**Patentanwälte Rechtsanwalt**
**Partnerschaft mbB**
**Joseph-Wild-Straße 20**
**81829 München (DE)**

Remarks:
This application was filed on 12-04-2019 as a divisional application to the application mentioned under INID code 62.

(54) **ALKYLATION CATALYST COMPRISING CERIUM RICH RARE EARTH CONTAINING ZEOLITES AND A HYDROGENATION METAL**

(57) An improved alkylation process utilizing a solid-acid catalyst comprising a cerium rich rare earth containing zeolite and a hydrogenation metal is disclosed.

EP 3 527 644 A1

**Description**

BACKGROUND OF THE INVENTION

The term alkylation refers to the reaction of an alkylatable compound, such as an aromatic or saturated hydrocarbon, with an alkylation agent, such as an olefin. The reaction is of interest because it makes it possible to obtain, through the alkylation of isoparaffins such as isobutane with an olefin containing 2-6 carbon atoms, an alkylate which has a high octane number and which boils in the gasoline range. Unlike gasoline obtained by cracking heavier petroleum fractions such as vacuum gas oil and atmospheric residue, gasoline obtained by alkylation is essentially free of contaminants such as sulfur and nitrogen and thus has clean burning characteristics. Its high anti-knock properties, represented by the high octane number, lessen the need to add environmentally harmful anti-knock compounds such as aromatics or lead. Also, unlike gasoline obtained by reforming naphtha or by cracking heavier petroleum fractions, alkylate contains few if any aromatics or olefins, which offers further environmental advantages.

The alkylation reaction is acid-catalyzed. Conventional alkylation process equipment makes use of liquid acid catalysts such as sulfuric acid and hydrofluoric acid. The use of such liquid acid catalysts is attended with a wide range of problems. For instance, sulfuric acid and hydrofluoric acid are both highly corrosive, so that the equipment used has to meet severe service requirements. Since the presence of highly corrosive materials in the resulting fuel is objectionable, the remaining acid must be removed from the alkylate. Also, because of the liquid phase separations that must be carried out, the process is complicated and expensive. In addition, there is always the risk that toxic substances such as hydrogen fluoride will be emitted to the environment.

Historically the activity and stability of solid acid alkylation catalysts have left much still to be desired when compared to competitive liquid acid alkylation processes. Recent developments in solid acid alkylation have included alkylation processes employing the facile regeneration of zeolite-containing solid acid catalysts, as disclosed in WO/9823560 (U.S. Pat. No. 5,986,158), improved solid acid catalyst production processes as per US Patent Application Publication 2007/0293390, alkylation catalyst hydration processes as per WO 2005/075387, continuous or semi-continuous alkylation and regeneration processes as per U.S. Pat. No. 7,176,340, US 2002/198422 and EP 1485334, and rare earth (RE) exchanged solid acid catalysts, as taught in U.S. Patent Application Publication 2008/0183025,

Another historical attempt at creating an active and stable solid acid alkylation catalyst includes U.S. Patent No. 3,851,004. The '004 reference elates to a process for the alkylation of hydrocarbons using zeolite-containing catalysts and more particularly to aromatic or isoparaffin alkylation processes wherein the reaction is catalyzed by a zeolitic molecular sieve catalyst in conjunction with a group VIII metal hydrogenation agent. However, the '004 reference specifically teaches that the addition of rare earth cations is not essential.

Other prior art attempts at creating an active and stable solid acid alkylation catalyst include U.S. Patent No. 8,163,969, U.S. Patent Application Publication 2010/0234661, and U.S. Patent Application Publication 2011/0313227. These references are herein incorporated by reference. These prior attempts disclose rare earth exchanged molecular sieves (e.g., Y-zeolites) in such solid acid alkylation catalysts.

There remains a need for a stable and active solid acid alkylation catalyst. The present invention provides an improved alkylation process utilizing a solid-acid catalyst comprising a cerium rich rare earth containing zeolite and a hydrogenation metal.

BRIEF DESCRIPTION OF THE INVENTION

It has been discovered that the use of cerium rich rare earth containing exchanged molecular sieves (e.g., Y-zeolites) in such solid acid alkylation catalysts, compared to low in cerium rare earth provided improved alkylation activity and stability of the catalyst.

In one embodiment of the invention there is provided a solid catalyst comprising a hydrogenation metal and a solid acid in the form of a cerium rich rare earth containing molecular sieve, wherein the catalyst is at least characterized by a porosity of less than 0.20 ml/g in pores below 100 nm in diameter, and a total porosity of greater than 0.30 ml/g.

Another embodiment of the invention provides a process for the alkylation of hydrocarbons comprising contacting a saturated hydrocarbon feedstock and one or more olefins with a catalyst of this invention at alkylation process conditions.

These and still further embodiments, features and advantages of the invention shall be made even more apparent by the followed detailed description, including the appended figures and claims.

BRIEF DESCRIPTION OF THE FIGURES

Figure 1 is a graph of olefin conversion over time

Figure 2 is a graph of C9+ compounds production over time

Figure 3 is a graph of olefin conversion over time

Figure 4 is a graph of C9+ compounds production over time

DETAILED DESCRIPTION OF THE INVENTION

All weight percentages mentioned related to the catalyst composition are based on dry catalyst (heated at 600°C for 1 hour). The rare earth wt% are calculated as rare earth oxides on a dry basis (600°C, 1 hour)

The water content of the catalyst ranges from about 1.5 wt% to about 6 wt%, in one embodiment it ranges from about 1.8 wt% to about 4 wt%, and in another embodiment it ranges from about 2 wt% to about 3 wt%. The water content of the catalyst is defined as its water content during use in the alkylation process and is measured by determining the water loss upon heating the catalyst to 600 °C including two hours at 600°C (LOI 600).

The catalyst further comprises a hydrogenation metal. Examples of suitable hydrogenation metals are the transition metals, such as metals of Group VIII of the Periodic Table, and mixtures thereof. Among these, noble metals of Group VIII of the Periodic Table are preferred. Platinum, palladium, and mixtures thereof are especially preferred. The amount of hydrogenation metal will depend on its nature. When the hydrogenation metal is a noble metal of Group VIII of the Periodic Table, the catalyst generally will contain in the range of about 0.01 to about 2 wt% of the metal. In one embodiment it ranges from about 0.1 to about 1 wt%, calculated as metal and based on the total weight of the catalyst.

The catalyst further comprises a solid acid. Examples of solid acids are zeolites such as zeolite beta, MCM-22, MCM-36, mordenite, faujasites such as X-zeolites and Y-zeolites, including H-Y-zeolites and USY-zeolites, non-zeolitic solid acids such as silica-alumina, sulfated oxides such as sulfated oxides of zirconium, titanium, or tin, mixed oxides of zirconium, molybdenum, tungsten, phosphorus, etc., and chlorinated aluminium oxides or clays. Preferred solid acids are zeolites, including mordenite, zeolite beta, faujasites such as X-zeolites and Y-zeolites, including HY-zeolites and USY-zeolites. Mixtures of solid acids can also be employed. In one embodiment the solid acid is a faujasite with a unit cell size ($a_0$) of 24.72 to about 25.00 angstroms, in another embodiment the solid acid is Y-zeolite with a unit cell size of 24.34 - 24.72 angstroms, while in another the solid acid is Y-zeolite with a unit cell size of 24.42 - 24.56 angstroms. In yet another embodiment the solid acid is Y-zeolite with a unit cell size of 24.56 - 24.72 angstroms.

The catalyst comprises rare earth, i.e., an element chosen from the lanthanide series or combinations of such elements. In one embodiment, the solid acid component of the catalyst comprises from about 0.5 wt % to about 32 wt % rare earth. In another, the solid acid component of the catalyst comprises from about 2 wt % to about 9 wt % rare earth. In yet another, the solid acid component of the catalyst comprises from about 4 wt% to about 6 wt% rare earth.

Further, at least a portion of the rare earth element component of the catalyst should be cerium. The amount of cerium in the final catalyst should be more than 0.1 wt%. More preferably, the cerium content should be more than 0.3 wt%. Most preferably, the cerium content should be at least 0.5%. The rare earth element(s) may be exchanged into the solid acid component by conventional means. In one embodiment, the rare earth element of the solid acid component is substantially all cerium. In another embodiment, the rare earth element of the solid acid component is a cerium rich rare earth mixture. In this mixture, the amount of cerium should be more than 3 wt% of the mixture. More preferably, the cerium content should be more than 5wt% of the mixture. Most preferably, the cerium content should be at least 10 % of the mixture. The balance of the rare earth mixture would substantially comprise one or more other rare earth elements, i.e., an element chosen from the lanthanide series, such as lanthanum, or combinations of such elements.

In other embodiments, additional cerium is added to the catalyst. This is performed by impregnation and/or ion exchange of the solid acid-containing particles. For example, this process can be carried out by pore volume impregnation using a cerium nitrate or cerium chloride solution and about 95-115wt%, preferably 105 wt% saturation level compared to the water pore volume of the catalyst. Followed by calcination at about 380-550°C, preferably 420-500°C. Preferably, the catalyst is dried, preferably at about 110-150°C, more preferably 120-130°C, before calcination. Alternatively, the catalyst particles may be exchanged with the cerium solution and dried and calcined at similar conditions as used after impregnation. Preferably, ion exchange and/or impregnation with cerium are carried out before addition of the group VIII metal(s) to the catalyst.

During the exchange process of the solid acid component, sodium (Na+) is removed from the catalyst. In one embodiment the solid acid component contains less than 1.5 wt% $Na_2O$. In another, less than 1.0 wt% $Na_2O$. In yet another less than 0.6 wt% $Na_2O$, all calculated on dry basis (600°C, 1 hour).

The catalyst may additionally comprise a matrix material. Examples of suitable matrix materials are alumina, silica, titania, zirconia, clays, and mixtures thereof. Matrix materials comprising alumina are generally preferred. In one embodiment, the catalyst comprises about 2 wt% to about 98 wt% of the solid acid and about 98 wt% to about 2 wt% of the matrix material, based on the total weight of the solid acid and the matrix material present in the catalyst. In another embodiment, the catalyst comprises about 10 wt% to about 90 wt% of the solid acid and about 90 wt% to about 10 wt% of the matrix material, based on the total weight of the solid acid and the matrix material contained in the catalyst. In another embodiment, the catalyst comprises about 10 wt% to about 80 wt% of matrix material and balance solid acid. In yet another embodiment, the catalyst comprises about 10 wt% to about 40 wt% of the matrix material and balance solid acid, based on the total weight of the solid acid and the matrix material contained in the catalyst

The catalyst preferably contains less than 0.5% wt of halogens. More preferably the catalyst contains no more than trace amounts of halogens.

The pore volume for pores less than 100 nm in diameter, as well as the total pore volume of produced catalysts were determined via mercury (Hg) intrusion on the basis of the Washburn equation

$$D = \frac{-4\gamma\cos\theta}{p}$$

with D being the pore diameter, p being the pressure applied during the measurement, $\gamma$ being the surface tension, taken to be 480 dynes/cm, and $\theta$ being the contact angle, taken to be 140°. In the present measurement, the pressure was varied over such a range that the measurement covered pores with a diameter in the range of 4.2 -8000 nm.

In one embodiment, the catalyst has a total pore volume of at least about 0.23 ml/g and in another at least about 0.25 ml/g. More preferably, the total pore volume is at least 0.3 ml/g and most preferably at least 0.4 ml/g.

The particles of the catalyst can have many different shapes, including spheres, cylinders, rings, and symmetric or asymmetric polylobes, for instance tri- and quadrulobes.

In one embodiment, the catalyst particles have an average particle diameter of at least about 0.5 mm, in another embodiment at least about 0.8 mm, and in yet another embodiment at least about 1.0 mm. In one embodiment, the upper limit of the average particle diameter lies at about 10.0 mm, in another at about 5.0 mm, and in yet another embodiment at about 3.0 mm.

Preferably, the catalyst consists essentially of a hydrogenation metal, a cerium rich rare earth exchanged molecular sieve and, optionally, a matrix material. More preferably, the catalyst consists essentially of one or more cerium rich rare earth exchanged faujasite(s), one or more Group VIII noble metal(s), and one or more matrix material(s). Even more preferably, the catalyst of the invention consists essentially of one or more Group VIII noble metal compounds, one or more cerium rich rare earth exchanged Y-zeolites, and one or more matrices comprising alumina.

The catalyst can be prepared by processes now known to the industry, modified to achieve the particular pore characteristics of this invention. A typical process comprises the successive steps of

(i) shaping, e.g., extruding the solid acid constituent, optionally after mixing it with a matrix material, to form particles,
(ii) calcining the resulting particles, and
(iii) incorporating the hydrogenation metal into the calcined particles by, e.g., impregnating the particles with a solution of a hydrogenation metal component and/or by (competitive) ion exchange.

Alternatively, the catalyst can, e.g., be prepared by a process comprising the successive steps of

(i) incorporating the hydrogenation metal into the solid acid constituent or into a mixture of the solid acid constituent and the matrix material,
(ii) shaping, e.g., extruding the resulting material to form particles, and
(iii) calcining the resulting particles.

With regard to catalyst preparation, the procedures described in US 2008183025 also can be followed. In order to obtain the particular porosity characteristics of the present invention, it is particularly useful to carry out the extrusion step carefully. Thus, it is particularly useful to carry out the extrusion as follows:

1) mixing the matrix material (e.g., precipitated alumina powder), rare earth-exchanged molecular sieve (e.g., zeolite), water, nitric acid and a few percent of an extrusion aid (e.g. methylcellulose) to form a mixture,
2) feeding this mixture to an extruder, and
3) depending on visual inspection of the resulting extrusion product, adding some extra water during extrusion.

In carrying out this procedure experimentally to obtain catalysts of the invention, it was observed that water content (LOI 600) of the final extrusion mixture was in the order of 45 to 55 wt %. In the order of 0.05 to 0.25 equivalent (relative to the alumina powder) of nitric acid was added. Zeolite content of the extrudates was in the order of 65 to 85 wt % and the balance matrix and hydrogenation metal (0.05 to 0.5 wt % Pt), calculated on dry basis (600° C, 1 hour). Those skilled in the art can now appreciate that the exact water content and acid addition required to get the extrudates with the desired properties (including physical strength such as side crushing strength and bulk crushing strength) depend on the molecular sieve content and the specific properties of the matrix material used. This is typically found by trial and error experiments after the starting component materials have been determined. The average particle length ranges from about 1 to about 6 mm, the particle diameter ranges from about 0.5 to about 3 mm, and the side crushing strength ranges from about 1 to about 10 lbs/mm.

The catalyst is particularly suitable for the alkylation of saturated hydrocarbons. The invention therefore further pertains to the use of the catalyst of the invention in the alkylation of these feedstocks. As stated above, this comprises the reaction of a saturated hydrocarbon with an olefin or olefin precursor in the presence of the catalyst of the invention to give highly branched saturated hydrocarbons with a higher molecular weight.

The hydrocarbon to be alkylated in the alkylation process is a branched saturated hydrocarbon such as an isoalkane having 4-10 carbon atoms. Examples are isobutane, isopentane, isohexane or mixtures thereof. The alkylation agent is an olefin or mixture of olefins having 2-10 carbon atoms. In one embodiment, the alkylation process consists of the alkylation of isobutane with butenes.

As will be evident to the skilled person, the alkylation process can take any suitable form, including fluidized bed processes, slurry processes, and fixed bed processes. The process can be carried out in a number of beds and/or reactors, each with separate addition of alkylation agent if desirable. In such a case, the process of the invention can be carried out in each separate bed or reactor.

As mentioned above, water may be added during the process in order to increase the water content of the catalyst to the desired level. This water can be introduced during the alkylation reaction via, e.g., the hydrocarbon feed or the feed of alkylation agent. Alternatively, the catalyst can be hydrated by using a water-containing atmosphere during the optional (mild) regeneration steps described below, or by contacting the catalyst with water in a separate intermediate hydration step. Similar procedures can be applied to rehydrate the catalyst after its water content has decreased during processing (i.e. during the alkylation reaction and/or regeneration).

The catalyst used in the process according to the invention is prepared by adjusting the water content. For example, the solid acid constituent may be mixed with a matrix material, to form carrier particles, followed by calcination of the particles. The hydrogenating function may, e.g., be incorporated into the catalyst composition by impregnating the carrier particles with a solution of a hydrogenation metal component. After impregnation the catalyst may be calcined.

In one embodiment, the catalyst is reduced at a temperature in the range of about 200 to about 500°C in a reducing gas such as hydrogen. In another embodiment, the catalyst is reduced at a temperature in the range of about 250 to about 350°C. The reduction can be performed before adjustment of the water content, after addition of water to the catalyst and/or by using reduction as a way to adjust the water content. In one embodiment, the reduction is performed before adjustment of the water content. In another, the reduction is performed after drying the catalyst in a dry, non-reducing gas (such as nitrogen, helium, air, and the like).

The water content of the catalyst can be adjusted by various methods as described in PCT/EP2005/000929, which is incorporated by reference in its entirety. Such methods are exemplified below as methods 1, 2, and 3.

Method 1 involves increasing the water content of a catalyst by exposing the catalyst to water. This can be achieved by exposing the catalyst to a water-containing atmosphere, e.g., air at ambient conditions. Embodiments of this method include exposing a reduced catalyst to water until the desired water content is reached, exposing an unreduced catalyst to water until a water content above the desired level is reached, followed by reduction of the catalyst, thereby decreasing the water content to the desired level, exposing a reduced catalyst to water until a water content above the desired level is reached, followed by treatment of the catalyst in either an inert or a reducing atmosphere, thereby decreasing the water content to the desired level, and reducing the catalyst in a hydrogen and water-containing atmosphere.

Method 2 involves decreasing the water content of an existing catalyst to the desired level by reducing an unreduced catalyst with a water content above the desired level.

Method 3 involves in-situ water addition by starting the alkylation process with a catalyst having a water content below the desired level and adding water to the alkylation unit during processing, for instance by adding water to the hydrocarbon feed, by regenerating the catalyst in a water-containing atmosphere and/or by exposing the regenerated catalyst to a water-containing atmosphere.

A combination of two or more of the above methods may also be employed.

Suitable process conditions are known to the skilled person. Preferably, an alkylation process as disclosed in WO 98/23560 is applied. The process conditions applied in the present process are summarized in the following Table:

|  | Temperature range [°C] | Pressure range [bar] | Molar ratio of hydrocarbon to alkylation agent |
|---|---|---|---|
| Preferred | -40 - 250 | 1 - 100 | 5:1 - 5,000:1 |
| More preferred | 20 - 150 | 5 - 40 | 50:1 - 1,000: |

(continued)

| | Temperature range [°C] | Pressure range [bar] | Molar ratio of hydrocarbon to alkylation agent |
|---|---|---|---|
| Most preferred | 65 - 95 | 15 - 30 | 150:1 - 750:1 |

Optionally, the catalyst may be subjected to high-temperature regeneration with hydrogen in the gas phase. This high-temperature regeneration may be carried out at a temperature of at least about 150°C, in one embodiment regeneration is carried out at about 150° to about 600°C, and another at about 200° to about 400°C. For details of this regeneration procedure, reference is made to WO 98/23560, and in particular to page 4, lines 12-19, which is herein incorporated in its entirety by reference. The high-temperature regeneration can be applied periodically during the alkylation process. If as a result of high-temperature regeneration the water content of the catalyst has decreased to below the desired level, the catalyst may be rehydrated during the process in the ways described above.

In addition to the high-temperature regeneration treatment, a milder regeneration may be applied during the alkylation process, such as described in WO 98/23560, in particular page 9, line 13 through page 13, line 2, which is herein incorporated in its entirety by reference. During the alkylation process, the catalyst may be subjected intermittently to a regeneration step by being contacted with a feed containing a hydrocarbon and hydrogen, with said regeneration being carried out at about 90% or less of the active cycle of the catalyst in one embodiment, at 60% or less in another embodiment, at 20% or less in yet another embodiment, and at 10% or less in another embodiment. The active cycle of the catalyst is defined herein as the time from the start of the feeding of the alkylation agent to the moment when, in comparison with the alkylation agent added to the catalyst-containing reactor section, 20% of the alkylation agent leaves the catalyst-containing reactor section without being converted, not counting isomerization inside the molecule.

In one embodiment, the preparation of a catalyst of the present invention can comprise the steps of: a) calcining solid acid-containing particles at a temperature in the range of about 400 to about 575°C; b) incorporating a Group VIII noble metal into the calcined particles to form noble metal-containing particles; and c) calcining the noble metal-containing particles at a temperature in the range of about 350 to about 600°C. Alternatively, after a), additional cerium can be added to the catalyst by ion exchange and /or impregnation followed by drying and/or calcination. Thereafter, the noble metal is added.

Performance in alkylation reactions of catalysts of the present invention can be further improved if the calcination steps before and after incorporation of cerium and after the incorporation of hydrogenation component are conducted in a specific temperature window.

The solid acid-containing particles are calcined in step a) at a temperature in the range of about 400 to about 575°C, in another embodiment in the range of about 450 to about 550°C, and in yet another embodiment in the range of about 460 to about 500°C. The heating rate ranges from about 0.1 to about 100°C/min, and in one embodiment from about 0.5°C to about 50°C/min, and in another embodiment from about 1 to about 30°C/min. Calcination is conducted for about 0.01 to about 10 hrs, and in one embodiment for about 0.1 to about 5 hrs, and in another embodiment for about 0.5 to about 2 hrs. It may be conducted in an air and/or inert gas (e.g. nitrogen) flow. In one embodiment this gas flow is dry.

In another embodiment, the solid acid-containing particles are dried before being calcined. This drying may be conducted at a temperature of about 110 to about 150°C.

The calcination can be performed in any equipment, such as a fixed bed reactor, a fluidized bed calciner, and a rotating tube calciner.

A Group VIII noble metal is then incorporated into the calcined solid acid-containing particles in step b). In one embodiment, this is performed by impregnation or competitive ion exchange of the solid acid-containing particles using a solution comprising Group VIII noble metal ions and/or their complexes and (optionally) $NH4^+$ ions. In another embodiment, the Group VIII noble metals are platinum, palladium, and combinations thereof. In yet another embodiment, at least one of the Group VIII noble metals is platinum. Suitable Group VIII noble metal salts include nitrates, chlorides, and ammonium nitrates of the noble metals or their complexes (e.g. $NH_3$ complexes).

The resulting noble metal-containing particles are then calcined at a temperature in the range of 350-600°C in step c). In one embodiment, the particles are calcined at about 400 to about 550°C, and in another from about 450 to about 500°C. This temperature is may be reached by heating the particles by about 0.1 to about 100°C/min to the desired final value between about 350 and about 600°C. In one embodiment, they are heated by about 0.5 to about 50°C/min, in another by about 1 to about 30°C/min. Calcination may be conducted for about 0.01 to about 10 hrs, and in one embodiment for about 0.1 to about 5 hrs, and in another for about 0.5 to about 2 hrs. Calcination may be conducted in an air and/or inert gas (e.g. nitrogen) flow. In one embodiment this gas flow is dry.

Optionally, a separate drying step is applied between steps (b) and (c). Alternatively, the noble metal-containing particles are dried during the calcination step. Also optionally, a dwell of about 15-120 minutes is introduced at a temperature of about 200 to about 250°C.

After calcination step (c), the resulting catalyst particles may be reduced at a temperature range of about 200 to about 500°C, in one embodiment from about 250 to about 350°C, in a reducing gas such as hydrogen.

The use of the catalyst of the present invention in the above alkylation process results in a high olefin conversion (amount of olefin in the feed that is converted in the reaction), a high C5+ alkylate yield (weight amount of C5+ alkylate produced divided by the overall weight of olefin consumed) and a high octane number, while the amount of undesired C9+ by-products can be restricted and the catalyst's stability can thus be improved. For details in respect of these parameters, reference is made to WO 9823560.

The following examples are presented for purposes of illustration, and are not intended to impose limitations on the scope of this invention.

## EXAMPLES

**Testing No. 1:** First, a comparative "low Ce" catalyst was made. According to the procedures described in US 2011/0313227 and US 8,163,969 a zeolite was prepared with about 4wt% of a "low Ce" rare earth (RE) mixture. After extrusion of about 75wt% of this zeolite using an alumina matrix and impregnation with Pt according to procedures described in US 2011/0313227 the final catalyst contained about 0.1wt% Ce, 2.8wt% RE total and 0.2wt% Pt

This so called "low Ce" catalyst was tested according to the procedures described in US 2011/0313227 and compared with catalysts of the invention.

Next, a catalyst of the invention with zeolite of higher Ce content (Ce on zeolite) was made. According to the procedures described in US 2011/0313227 and US 8,163,969 a zeolite was prepared with about 4wt% of a "high Ce" rare earth mixture. After extrusion of about 75wt% of this zeolite using an alumina matrix and impregnation with Pt according to procedures described in US 2011/0313227 the final catalyst contained about 0.5wt% Ce, 3wt% RE total and 0.2wt% Pt. This so called "Ce on zeolite" catalyst was tested according to the procedures described in US 2011/0313227 and compared with the "low Ce" comparative catalyst.

Third, a catalyst of the invention with additional Ce impregnated was made. A sample of the "low Ce" catalyst extrudates of the comparative example taken before Pt impregnation was impregnated with an additional amount of Ce to increase the Ce content from about 0.1wt% to about 0.5wt%. Pore volume impregnation was carried out using a Ce nitrate solution and about 105 wt% saturation level compared to the water pore volume of the catalyst. The Ce impregnated catalyst was dried and calcined using similar procedures as described in US 2011/0313227 for the impregnation of Pt. Thereafter, Pt was impregnated also according to similar procedures as described in US 2011/0313227. The final catalyst contained about 0.5wt% Ce and about 0.2wt% Pt.

This so called "Ce impregnated" catalyst was tested according to the procedures described in US 2011/0313227 and compared with the "low" Ce comparative catalyst.

**Testing No. 2:** According to the procedures described in US 2011/031322 a zeolite was prepared with about 5wt% of a "high Ce" rare earth mixture. After extrusion of about 75wt% of this zeolite using an alumina matrix and impregnation with Pt according to procedures described in US 2011/0313227 the final catalyst contained about 1 wt% Ce, 3.8wt% RE total and 0.2wt% Pt. A second exemplar catalyst was made in a similar way and prepared with 2wt% Ce, 3.8wt% RE total and 0.2wt% Pt. Finally, a third exemplar catalyst was made using similar methods and was prepared with 4.8wt% of Ce only.

The tested alkylation catalysts from both Testing No. 1 and Testing No. 2 had the following compositions and properties: from about 60 to about 75% of the above-described zeolite, from about 25% to about 40% alumina, from about Pt 0.2wt% to about 0.5% platinum, the average particle length ranges from about 1 to about 6 mm, the average length/diameter ratio ranges from about 1 to about 12 , the particle diameter ranges from about 0.5 to about 3 mm, and the side crush strength ranges from about 1 to about 10 lbs/mm.

General Test Procedure:

A fixed-bed recycle reactor as described in WO 9823560, which is herein incorporated by reference in its entirety, having a diameter of 2 cm was filled with a 1:1 volume/volume mixture of 38.6 grams of catalyst extrudates (on dry basis, LOI600)and carborundum particles (60 mesh). At the center of the reactor tube a thermocouple of 6 mm in diameter was arranged. The reactor was flushed with dry nitrogen for 30 minutes (21 Nl/hour). Next, the system was tested for leakages at elevated pressure, after which the pressure was set to 21 bar and the nitrogen flow to 21 Nl/hour. The reactor temperature was then raised to 275°C at a rate of 1°C/min, at 275°C nitrogen was replaced by dry hydrogen and the catalyst was reduced at 275°C.

Alternatively, in case of high temperature regeneration of the same catalyst sample between runs, after draining and flushing the reactor with hydrogen to remove hydrocarbons while maintaining the alkylation reaction temperature, hydrogen flow was set to 21 Nl/hour and the reactor temperature was then raised to 275°C at a rate of 1°C/min, and the catalyst was regenerated at 275°C.

After 2 hours, the reactor temperature was lowered to the reaction temperature of about 75°C. During cooling down water was added to the hydrogen flow to obtain an water content of the catalyst of about 2-4 wt% (defined as the catalyst's water loss after heating for two hours at 600°C).

The hydrogen stream was stopped with the attaining of the reaction temperature. Isobutane containing about 4 wt% alkylate (added to accelerate deactivation rate, composition of the alkylate added is similar to alkylate produced by the process at the conditions described) and about 1 mol% of dissolved hydrogen was supplied to the reactor at a rate of about 4.0 kg/hour. About 95-98% of the isobutane/alkylate mixture was fed back to the reactor. About 2-5% was drained off for analysis. Such an amount of isobutane/alkylate mixture was supplied to the reactor as to ensure a constant quantity of liquid in the system. When the system had stabilized, hydrogen addition was stopped and such an amount of cis-2-butene was added to it as to give a cis-2-butene-WHSV of 0.16. The overall rate of flow of liquid in the system was maintained at about 4.0 kg/h. The weight ratio of isobutane to cis-2-butene at the reactor inlet was about 600 - 700. The pressure in the reactor amounted to about 21 bar. Total alkylate concentration of the hydrocarbon recycle flow (from added and produced alkylate) was maintained at about 8-9 wt% during the test by controlling the drain off flow to analyses.

Each time after 1 hour of reaction, the catalyst was regenerated by being washed with isobutane/alkylate mixture for 5 minutes, followed by 50 minutes of regeneration through being contacted with a solution of 1 mole% of H2 in isobutane/alkylate mixture, and then being washed with isobutane/alkylate mixture for another 5 minutes (total washing and regeneration time 1 hour). After this washing step, alkylation was started again.

The temperature during the washing steps, the regeneration step, and the reaction step was the same.

The process was conducted as above and the catalytic performance was measured as a function of time. The performance was characterized by the olefin conversion per reactor pass. Olefin conversion per reactor pass is the weight fraction (as a percentage) of olefins that is converted between the inlet - and the outlet of the catalyst bed, not counting isomerization within the olefin molecules.

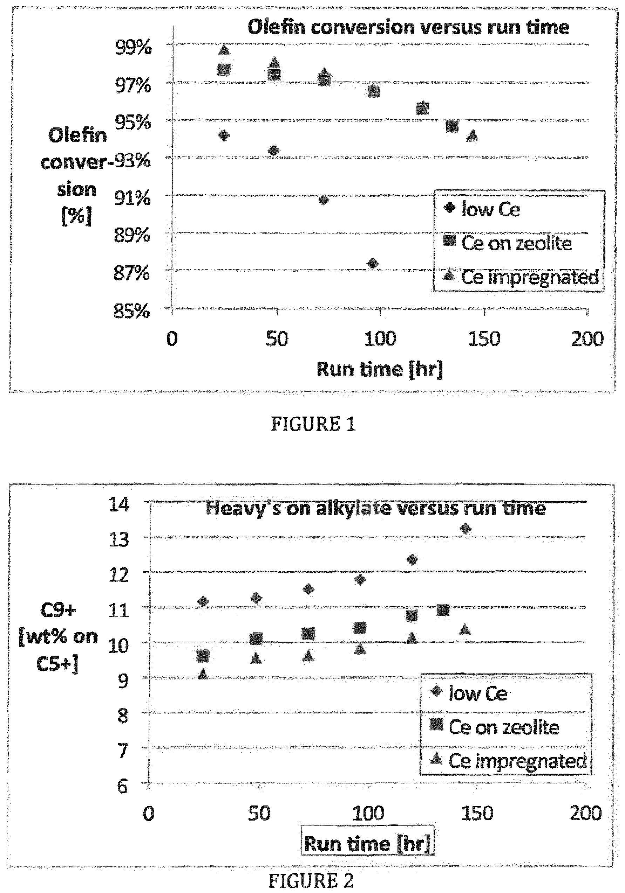

Results obtained with the various catalysts of Testing No. 1 are presented in Figures 1 and 2. Figure 1 shows olefin conversion versus run time. Figure 2 shows C9+ compounds (heavy's) versus run time.

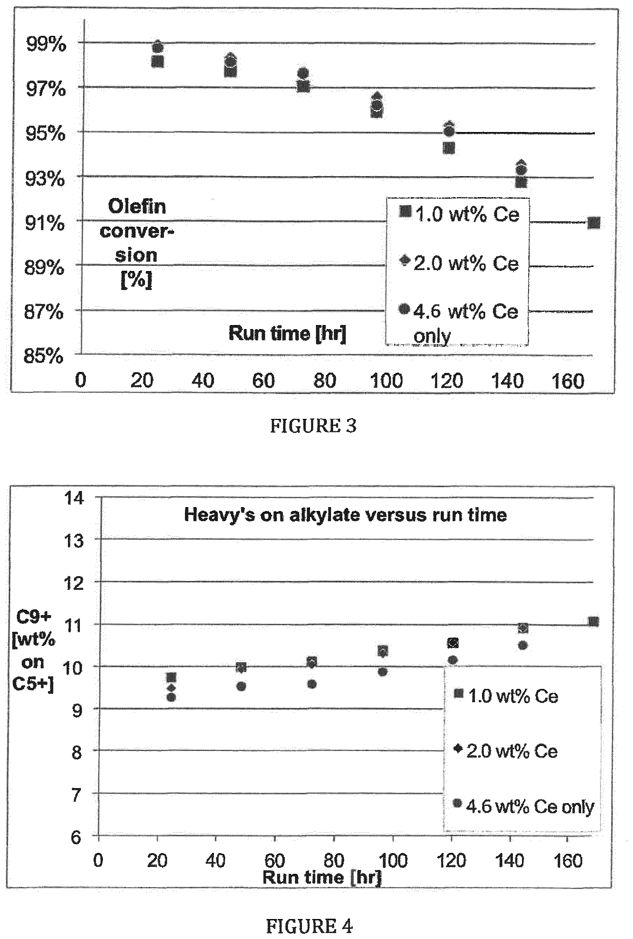

Results obtained with the various catalyst of Testing No. 2 are presented in Figures 3 and 4. From these results, it can be concluded that the samples with higher than 0.5wt% Ce give similar results

The invention also relates the following numbered embodiments

1. A process for alkylating hydrocarbons wherein an alkylatable organic compound is reacted with an alkylation agent to form an alkylate in the presence of a catalyst with the catalyst being subjected intermittently to a regeneration step by being contacted with a feed containing a saturated hydrocarbon and hydrogen, said regeneration being carried out at 90% or less of the active cycle of the catalyst, with the active cycle of the catalyst being defined as the time from the start of the feeding of the alkylation agent to the moment when, in comparison with the entrance of the catalyst-containing reactor section, 20% of the alkylation agent leaves the catalyst-containing reactor section without being converted, not counting isomerization inside the molecule, wherein said catalyst comprises a hydrogenating function, solid acid constituent and one or more rare earth elements, said one or more rare earth elements comprising at least 0.1 wt % cerium calculated as fraction of the total catalyst weight.

2. A process according to embodiment 1, wherein the one or more rare earth elements comprise at least 0.3 wt % cerium, calculated as fraction of the total catalyst weight.

3. A process according to embodiment 1, wherein the one or more rare earth elements comprise at least 0.5 wt % cerium calculated as fraction of the total catalyst weight.

4. A process according to embodiment 1, wherein the one or more rare earth elements are comprised of cerium and lanthanum.

5. A process according to embodiment 1, wherein the cerium is added to the catalyst and/or the solid acid constituent either by impregnation or by ion exchange or a combination of the two.

6. The process of embodiment 1 wherein the alkylatable organic compound comprises an isoparaffin or mixture of isoparaffins and the alkylation agent comprises C3-C5 alkenes or mixture of C3-C5 alkenes

7. The process of embodiment 6 wherein the alkylation agent is n-butene or a mixture of butenes.

8. The process of embodiment 6 wherein the alkylatable organic compound is isobutane

9. The process of embodiment 1 wherein the catalyst comprises a hydrogenation function on a carrier comprising 2-98 wt. % of matrix material and the balance solid acid constituent.

10. The process of embodiment 9 wherein the catalyst carrier comprises 20-80 wt. % of matrix material and the balance solid acid constituent.

11. The process of embodiment 10, wherein the catalyst carrier comprises 20-50 wt. % of matrix material and the balance solid acid constituent.

12. The process of embodiment 9 wherein the matrix material comprises alumina.

13. The process of embodiment 1 wherein the solid acid constituent is a faujasite or zeolite beta.

14. The process of embodiment 13 wherein the solid acid constituent is a Y-zeolite.

15. The process of embodiment 14 wherein the solid acid constituent is prepared by a process comprising the steps

EP 3 527 644 A1

of: preparing a sodium zeolite, ion exchanging the sodium zeolite with $NH_4^+$ - and/or rare earth ions to reduce $Na_2O$ to about 3 - 6 wt%, steaming the zeolite at about 400 to about 500°C such that ao ranges from about 24.56 to about 24.72Å, ion exchanging with $NH_4^+$ ions to reduce $Na_2O$ to below about 1.5 wt%, and drying.

16. The process of embodiment 14 wherein the solid acid constituent is prepared by a process comprising the steps of: preparing a sodium zeolite, ion exchanging the sodium zeolite with $NH_4^+$ - and/or rare earth ions to reduce $Na_2O$ to about 3.5 - 4.5 wt%, steaming the zeolite at about 400 to about 500°C such that $a_0$ ranges from about 24.58 to about 24.68 Å, ion exchanging with $NH_4^+$ ions to reduce $Na_2O$ to below about 1.0 wt%, and drying.

17. The process of embodiment 14 wherein the solid acid constituent is prepared by a process comprising the steps of: preparing a sodium zeolite, ion exchanging the sodium zeolite with $NH_4^+$ - and/or rare earth ions to reduce $Na_2O$ to about 3.5 - 4.5 wt%, steaming the zeolite at about 400 to about 500°C such that ao ranges from about 24.60 to about 24.66 Å, ion exchanging with $NH_4^+$ ions to reduce $Na_2O$ to below about 0.7 wt%, and drying.

18. The process of embodiment 16 wherein the hydrogenation function is platinum, palladium, or a mixture thereof.

19. The process of embodiment 1 wherein the catalyst is prepared by a) calcining solid acid-containing particles at a temperature in the range of 400-575°C, b) incorporating a Group VIII noble metal into the calcined particles to form noble metal-containing particles, and c) calcining the noble metal-containing particles at a temperature in the range of 350-600°C.

20. A process according to embodiment 19 wherein the temperature applied in step a) is in the range of 450-550°C.

21. A process according to embodiment 20 wherein the temperature is in the range of 460-500°C.

22. A process according to embodiment 21 wherein the temperature applied in step c) is in the range of 400-550°C.

23. A process according to embodiment 22 wherein the temperature is in the range of 450-500°C.

24. A process according to embodiment 23 wherein the solid acid is a zeolite selected from the group consisting of zeolite beta and faujasites.

25. The process according to embodiment 1 wherein the catalyst further comprises from about 1.5 to about 6 wt% of water (LOI600).

26. The process according to embodiment 25 wherein the catalyst comprises from about 1.8 to about 4 wt% of water(LOI600).

27. The process according to embodiment 25 wherein the catalyst comprises from about 2 to about 3 wt% of water(LOI600).

28. The process according to embodiment 26 wherein the solid acid is a faujasite or zeolite beta.

29. The process according to embodiment 28 wherein the solid acid is Y-zeolite.

30. The process according to embodiment 29 wherein the hydrogenation metal is a Group VIII noble metal.

31. The process according to embodiment 25 wherein the catalyst is prepared by adding water to a dry catalyst comprising solid acid and hydrogenation metal before use in the alkylation process.

32. The process according to embodiment 25 wherein the alkylation process is started using a catalyst comprising less than about 1.5 wt% water and wherein water is added to the catalyst during the alkylation process.

33. An alkylating catalyst comprising:

a. hydrogenation metal in the amount of 0.05 to 0.5 wt %
b. solid acid constituent in the form of a rare earth exchanged molecular sieve with 2-9 wt % of one or more rare earth elements, wherein the one or more rare earth elements comprises at least 5 wt % cerium calculated on the total of rare earth elements.

34. The catalyst according to embodiment 33 wherein the solid acid constituent comprises a zeolite.

35. The catalyst according to embodiment 34 wherein the zeolite comprises a zeolite having a faujasite structure.

36. The catalyst according to embodiment 35 wherein the zeolite is Y-zeolite.

37. The catalyst according to embodiment 36, wherein the Y-zeolite has a unit cell size in the range of 24.56-24.72 angstroms.

38. The catalyst according to embodiment 37, wherein the unit cell size is in the range of 24.62-24.70 angstroms.

39. The catalyst according to embodiment 33, wherein the solid acid comprises no more than about 1 wt % Na2O, calculated on a dry basis (600° C., 1 hour).

40. The catalyst according to embodiment 39, wherein the solid acid comprises no more than about 0.8 wt % Na2O calculated on a dry basis (600° C., 1 hour).

41. The catalyst according to embodiment 33, wherein the hydrogenation metal consists essentially of a Group VIII noble metal.

42. The catalyst according to embodiment 41, wherein the Group VIII noble metal is platinum.

43. The catalyst according to embodiment 33, wherein the catalyst additionally comprises a matrix material.

44. The catalyst according to embodiment 43, wherein the matrix material comprises alumina.

45. The catalyst according to embodiment 33, wherein the catalyst further comprises an amount of water in the range of about 1.5 to about 6 wt %.

46. The catalyst according to embodiment 45, wherein the amount of water is in the range of about 2 to about 4 wt %.

**Claims**

1. An alkylating catalyst comprising:

   a. hydrogenation metal in the amount of 0.05 to 0.5 wt %

   b. solid acid constituent in the form of a rare earth exchanged molecular sieve with 2-6 wt % of one or more rare earth elements, wherein the one or more rare earth elements comprises at least 5 wt % cerium calculated on the total of rare earth elements, wherein the solid acid constituent preferably comprises a zeolite, more preferably wherein the zeolite comprises a zeolite having a faujasite structure, even more preferably wherein the zeolite is Y-zeolite, in particular wherein the Y-zeolite has a unit cell size in the range of 24.56-24.72 angstroms, preferably in the range of 24.62-24.70 angstroms; wherein the cerium content in the catalyst is more than 0.3 wt %.

2. The catalyst according to claim 1, wherein the solid acid comprises no more than about 1 wt %, preferably no more than about 0.8 wt % $Na_2O$ calculated on a dry basis (600° C., 1 hour).

3. The catalyst according to claim 1, wherein the hydrogenation metal consists essentially of a Group VIII noble metal, preferably wherein the Group VIII noble metal is platinum.

4. The catalyst according to claim 1, wherein the catalyst additionally comprises a matrix material, preferably wherein the matrix material comprises alumina.

5. The catalyst according to claim 1, wherein the catalyst further comprises an amount of water in the range of 1.5 to 6 wt %, preferably in the range of 2 to 4 wt %.

6. Use of an alkylating catalyst in a process for alkylating hydrocarbons comprising contacting a saturated hydrocarbon feedstock and one or more olefins with an alkylating catalyst as defined in any one of claims 1 to 5.

7. Use of an alkylating catalyst as defined in any one of claims 1 to 5 for achieving improved alkylation activity and stability of the catalyst.

FIGURE 1

FIGURE 2

FIGURE 3

FIGURE 4

Europäisches
Patentamt

European
Patent Office

Office européen
des brevets

# EUROPEAN SEARCH REPORT

Application Number

EP 19 16 8881

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 300 015 A (KIRSCH FRANCIS W ET AL) 10 November 1981 (1981-11-10) <br> * column 2, lines 48-53 * <br> * column 7, lines 12-38 * <br> * column 28, lines 60-65 * <br> * claim 1 * <br> ----- | 1-7 | INV. <br> C10G29/20 <br> B01J29/08 <br> C07C2/58 <br> B01J35/02 <br> B01J38/56 <br> B01J38/58 |
| A | US 2013/029832 A1 (LAI WENYIH FRANK [US] ET AL) 31 January 2013 (2013-01-31) <br> * the whole document * <br> * paragraphs [0044], [0046] - [0048] * <br> * paragraphs [0052], [0053] * <br> * paragraphs [0055], [0056], [0058], [0059], [0061] * <br> * paragraphs [0062], [0063] * <br> * paragraphs [0074], [0086], [0092] * <br> ----- | 1-7 | B01J29/12 <br> B01J29/70 <br> B01J29/90 <br> B01J29/068 <br> B01J29/74 |
| A | US 2013/180884 A1 (MINOUX DELPHINE [BE] ET AL) 18 July 2013 (2013-07-18) <br> * the whole document * <br> * paragraphs [0067], [0069] * <br> * paragraphs [0072] - [0074] * <br> ----- | 1-7 | |

TECHNICAL FIELDS
SEARCHED      (IPC)

C10G
B01J
C07C

The present search report has been drawn up for all claims

| Place of search | Date of completion of the search | Examiner |
|---|---|---|
| The Hague | 22 May 2019 | Iyer-Baldew, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 .................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 16 8881

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2019

| Patent document cited in search report | | Publication date | Patent family member(s) | Publication date |
|---|---|---|---|---|
| US 4300015 | A | 10-11-1981 | NONE | |
| US 2013029832 | A1 | 31-01-2013 | NONE | |
| US 2013180884 | A1 | 18-07-2013 | BR 112013000538 A2 | 24-05-2016 |
| | | | CN 103097493 A | 08-05-2013 |
| | | | EP 2404980 A1 | 11-01-2012 |
| | | | EP 2591074 A1 | 15-05-2013 |
| | | | KR 20130069724 A | 26-06-2013 |
| | | | US 2013180884 A1 | 18-07-2013 |
| | | | WO 2012004328 A1 | 12-01-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9823560 A
- US 5986158 A
- US 20070293390 A
- WO 2005075387 A
- US 7176340 B
- US 2002198422 A
- EP 1485334 A
- US 20080183025 A

- US 3851004 A
- US 8163969 B
- US 20100234661 A
- US 20110313227 A
- US 2008183025 A
- EP 2005000929 W
- US 8163969 A
- US 2011031322 A